Europäisches Patentamt

(19) European Patent Office

Office européen des brevets

(11) Numéro de publication: **0 058 623**
A1

# (12) DEMANDE DE BREVET EUROPEEN

(21) Numéro de dépôt: 82400273.7

(51) Int. Cl.³: **A 61 F 1/00**

(22) Date de dépôt: 16.02.82

(30) Priorité: 18.02.81 FR 8103220

(43) Date de publication de la demande:
25.08.82 Bulletin 82/34

(84) Etats contractants désignés:
BE DE GB

(71) Demandeur: LABORATOIRES BIOTROL S.A.
1, rue du Foin
F-75140 Paris Cedex 03(FR)

(72) Inventeur: Maupetit, Philippe
23, rue François Massé
F-93600 Aulnay sous Bois(FR)

(74) Mandataire: Phélip, Bruno et al,
c/o Cabinet Harlé & Phélip 21, rue de La Rochefoucauld
F-75009 Paris(FR)

(54) Prothèse vasculaire et dispositif pour sa mise en place.

(57) Prothèse vasculaire consistant essentiellement en un manchon tubulaire portant sur sa surface latérale externe des parties en relief orientées et dont une fraction pouvant attendre la moitié est inversée par rapport aux autres.

Le dispositif pour sa mise en place comprend une âme centrale souple servant de guide, une gaine extérieure tubulaire souple et agencée à son extrémité distale de façon à retenir temporairement la prothèse préalablement enfilée sur l'âme centrale susdite, ainsi qu'un organe tubulaire souple coulissant entre l'âme centrale et la gaine et constituant une butée arrière assurant le maintien de la prothèse dans la partie distale dudit dispositif.

Application notamment à titre de prothèse des voies biliaires ou pour anastomose porto-cave.

EP 0 058 623 A1

"Prothèse vasculaire et dispositif pour sa mise en place"

La présente invention concerne une prothèse vasculaire, notamment utile comme prothèse des voies biliaires et comme prothèse pour anastomose porto-cave, et un dispositif pour l'introduction et la mise en place d'une telle prothèse.

Pour la clarté de l'exposé, l'invention est décrite ci-après en référence plus spécialement au cas d'une anastomose porto-cave, mais il est bien clair que l'invention ne saurait être limitée à cette application.

La présence de tissus hépatiques sclérosés chez les patients atteints de cirrhose du foie constitue un obstacle préjudiciable à la libre circulation des fluides dans et à travers le parenchyme hépatique.

La cirrhose constitue un processus anatomopathologique du foie, caractérisé par la mutilation du parenchyme hépatique. Cette sclérose entraîne une insuffisance hépatique et une hypertension portale par blocage.

On ne dispose pas à ce jour de moyen pour restaurer les cellules sclérosées dans leurs fonctions vitales et la seule solution actuellement disponible dans de telles situations consiste à pratiquer une intervention chirurgicale au cours de laquelle on réalise un shunt partiel de la circulation veineuse hépatique. Il est cependant connu que cela nécessite des interventions dont le caractère traumatique constitue une gêne sérieuse, surtout s'agissant de malades dont les fonctions vitales sont déjà notablement affectées et chez qui l'hypertension portale entraîne inévitablement des troubles circulatoires.

Le document FR-A-2.361.862 décrit une prothèse vasculaire composite, faite d'un tube poreux de PTFE, dont les pores sont remplis d'un polymère hydrosoluble rendu insoluble dans l'eau et dont les pores ont subi des arrangements ou déformations tels que la formation de caillots sanguins à leur abord s'en trouve inhibée.

Le document FR-A-2.339.387 décrit un greffon vasculaire synthétique réalisé en double velours tricoté, ayant sur sa périphérie aussi bien interne qu'externe des boucles destinées à accroître la vitesse de croissance de tissu pénétrant dans ce greffon.

Le document FR-A-2.257.262 décrit une prothèse vasculaire poreuse, de structure filamentaire et comportant des filaments (formant en particulier une boucle de velours) s'étendant respectivement vers l'extérieur et vers l'intérieur de la prothèse tubulaire, là encore pour favoriser la fixation à cette prothèse de tissu de péri-greffe.

On a maintenant trouvé et mis au point une prothèse vasculaire, ainsi qu'un dispositif pour l'introduction et la mise en place d'une telle prothèse, dont l'utilisation est extrêmement aisée et qui, grâce à une mise en place par voie percutanée, n'entraîne pas les effets secondaires nuisibles rappelés ci-dessus.

La présente invention a pour premier objet une prothèse vasculaire qui est caractérisée en ce qu'elle comprend essentiellement un manchon tubulaire portant sur sa surface latérale externe des parties en relief, telles que notamment des ergots ou des écailles, qui sont orientées, avec leur plan de symétrie parallèle à ou passant par l'axe de la prothèse, et dont une fraction pouvant atteindre la moitié est inversée par rapport aux autres.

Ladite prothèse possède en pratique un diamètre extérieur, une épaisseur de paroi et une longueur appropriés pour l'usage auquel on destine cette prothèse.

Selon un mode de réalisation avantageux, cette prothèse est constituée d'un manchon tubulaire sur la surface duquel ont été soulevées par entaille des écailles rabattables, dont une fraction pouvant atteindre la moitié est inversée par rapport aux autres. L'alternance du sens des écailles et le nombre de celles qui se trouvent orientées respectivement dans un sens ou dans l'autre ne sont pas déterminantes et peuvent être choisis à convenance.

Bien qu'une telle prothèse puisse être réalisée en un matériau quelconque, pour autant que celui-ci soit bien toléré par les tissus avec lesquels il entre en contact et ne se dégrade pratiquement pas au cours du temps une fois mis en place, on préfère tout particulièrement une prothèse en polytétrafluoroéthylène (matériau notamment commercialisé sous la dénomination Teflon par E.I. DuPont de Nemours), en silicones ou dérivés, en polyéthylène, en polyamide ou en polyuréthane, un polyuréthane étant tout particulièrement préféré.

Pour le choix des matériaux appropriés, l'homme de métier a seulement à effectuer des essais de routine, pour lesquels il doit prendre en considération le fait que le matériau doit avantageusement être non toxique, ne pas induire d'hémolyse, ne pas être biologiquement dégradable et ne pas induire de coagulation.

Grâce à la présence de telles parties en relief orientées, telles que des ergots ou écailles, la prothèse selon l'invention présente des aspérités dissymétriques qui assurent de façon simple et efficace son maintien en place par auto-blocage dans les vaisseaux ou les parenchymes où son utilisation s'avère utile.

Dans la forme de réalisation comportant des écailles rabattables, l'introduction de la prothèse est facilitée et celle-ci peut même avoir un diamètre aussi grand que nécessaire, puisque les écailles ne viennent pas en surépaisseur de la prothèse proprement dite et ne viennent donc pas gêner l'introduction et la mise en place de cette prothèse qui peut avoir la dimension optimale requise.Une fois la prothèse en place, les écailles assurent le blocage nécessaire grâce à l'élasticité du matériau.

La réalisation d'ergots ou d'écailles sur tout ou partie du pourtour de la prothèse peut s'effectuer par tout moyen connu de l'homme de l'art, tel par exemple par des entailles en biais appropriées, au moyen d'outils coupants, sur une profondeur qui est en rapport avec la dimension des écailles qu'on veut obtenir.

La présente invention a également pour objet un dispositif pour l'introduction et la mise en place d'une telle prothèse, comprenant fondamentalement une âme centrale souple servant de guide, une gaine extérieure tubulaire souple et agencée à son extrémité distale de façon à retenir temporairement la prothèse préalablement enfilée sur l'âme centrale susdite, ainsi qu'un organe tubulaire souple coulissant entre l'âme centrale et la gaine et constituant une butée arrière assurant le maintien de la prothèse dans la partie distale dudit dispositif.

Selon un mode de réalisation avantageux, la gaine extérieure tubulaire se termine à son extrémité distale, par un sertissage en étoile apte à s'évaser lorsqu'on retire brusquement la gaine une fois la prothèse amenée à l'endroit voulu.

Dans une variante préférée, ladite gaine du dispositif selon l'invention comprend également une partie proximale évasée, dont la liaison au reste de la gaine tubulaire est assurée par un rétreint et qui a une longueur suffisante pour pouvoir contenir une prothèse. Il suffit alors de pousser la prothèse au moyen de l'organe tubulaire qui va lui servir de butée arrière pour que, en particulier dans le cas où ladite prothèse comporte des écailles rabattables, celle-ci pénètre facilement dans la gaine tubulaire proprement dite.

Un tel dispositif doit être d'une longueur suffisante pour pouvoir atteindre la partie lésée de l'organisme à traiter tout en restant accessible de l'extérieur par son extrémité proximale.

En pratique, une longueur d'environ 60 à 100 cm convient pour la mise en place d'une prothèse pour anastomose porto-cave avec introduction du dispositif par la veine jugulaire et cheminement par la veine cave inférieure et la branche sus-hépatique.

Les dimensions et les épaisseurs des différentes parties de ce dispositif peuvent être, en liaison avec celles de la prothèse proprement dite, adaptées par l'homme de l'art en fonction du cas d'espèce considéré.

Les matériaux constituant les diverses parties de ce dispositif sont également choisis selon les convenances, les disponibilités et/ou les contraintes économiques que l'homme de l'art peut rencontrer. En pratique, on préfère tout particulièrement pour chaque partie de ce dispositif un polytétrafluoroéthylène tel que ceux qui sont commercialisés sous la dénomination Teflon, mais il est bien clair que tout autre matériau bien toléré par l'organisme du patient et ne se dégradant pas in situ peut également convenir.

Bien que l'invention ne soit pas limitée à cet application, on décrit ci-après la mise en place de la prothèse au moyen du dispositif selon l'invention dans le cas, purement illustratif, d'une anastomose porto-cave.

Pour réaliser une telle anastomose conformément à la présente invention, on pratique une ponction au niveau de la veine jugulaire du patient et on introduit dans celle-ci un dispositif permettant, grâce à une aiguille, une ponction hépatique mettant en communication la veine sus-hépatique où chemine le matériel avec une branche de la veine porte. L'âme-guide du dispositif selon l'invention est alors introduite de manière à être laissée en place dans le shunt ainsi réalisé. On fait alors glisser sur ce guide le reste du dispositif selon l'invention, contenant une prothèse appropriée telle que décrite plus haut, en suivant son cheminement, par exemple/radioscopie. On s'applique donc à faire passer celui-ci dans la veine sus-hépatique et, de là, dans une branche du système veineux sus-hépatique. On amène ensuite le dispositif jusque dans une branche correspondante du système veineux porte, de telle sorte que la prothèse se trouve en pontage entre les branches considérées respectivement de la veine porte et de la veine sus-hépatique. On retire alors brusquement la gaine du système, ce qui a pour effet de libérer la prothèse et de dégager les aspérités qu'elle comporte et qui viennent prendre appui sur les parois internes des branches veineuses concernées et du parenchyme situé entre elles. On retire ensuite complètement ladite gaine, ainsi que l'âme et l'organe tubulaire qui était destiné à assurer une butée arrière pour la prothèse comme indiqué plus haut.

## REVENDICATIONS

1. Prothèse vasculaire, caractérisée en ce qu'elle consiste essentiellement en un manchon tubulaire portant sur sa surface latérale externe des parties en relief orientées et dont une fraction pouvant atteindre la moitié est inversée par rapport aux autres.

2. Prothèse selon la revendication 1, caractérisée en ce que lesdites parties en relief sont des ergots ou des écailles.

3. Prothèse selon la revendication 1, caractérisée en ce qu'elle comprend des écailles rabattables, soulevées par entaille dudit manchon et dont une fraction pouvant atteindre la moitié est inversée par rapport aux autres.

4. Prothèse selon l'une quelconque des revendications 1 à 3, caractérisée en ce qu'elle est réalisée en polytétrafluoroéthylène, en silicone ou dérivés, en polyéthylène, en polyamide ou en polyuréthane.

5. Dispositif pour l'introduction et la mise en place d'une prothèse vasculaire selon la revendication 1, caractérisé en ce qu'il comprend fondamentalement une âme centrale souple servant de guide, une gaine extérieure tubulaire souple et agencée à son extrémité distale de façon à retenir temporairement la prothèse préalablement enfilée sur l'âme centrale susdite, ainsi qu'un organe tubulaire souple coulissant entre l'âme centrale et la gaine et constituant une butée arrière assurant le maintien de la prothèse dans la partie distale dudit dispositif.

6. Dispositif selon la revendication 5, caractérisé en ce que la gaine extérieure tubulaire se termine, à son extrémité distale, par un sertissage en étoile apte à s'évaser lorsqu'on retire brusquement la gaine une fois la prothèse amenée à l'-endroit voulu.

7. Dispositif selon l'une des revendications 5 ou 6, caractérisé en ce que la gaine comprend une partie proximale évasée, dont la liaison au reste de la gaine tubulaire est assurée par un rétreint et qui a une longueur suffisante pour pouvoir contenir une prothèse.

8. Dispositif selon l'une quelconque des revendications 5 à 7, caractérisé en ce que ces diverses parties sont réalisées en polytétrafluoroéthylène.

**Office européen des brevets**

**RAPPORT DE RECHERCHE EUROPEENNE**

Numéro de la demande

EP 82 40 0273

| Catégorie | Citation du document avec indication, en cas de besoin, des parties pertinentes | Revendication concernee |
|---|---|---|
| DA | FR - A - 2 361 862 (SUMITOMO ELECTRIC INDUSTRIES LTD)<br><br>* Revendications 1-7 *<br><br>-- | 1,4 |
| DA | FR - A - 2 339 387 (MEADOX MEDI-CALS INC.)<br><br>* Page 5, ligne 18 - page 6, ligne 5; figures 1,2 *<br><br>-- | 1,2 |
| DA | FR - A - 2 257 262 (SAUVAGE)<br><br>* Page 4, lignes 6-17; page 10, ligne 25 - page 11, ligne 3; figures 1,3 *<br><br>-- | 1,2,4 |
| A | FR - A - 2 435 953 (O'NEIL)<br><br>* Page 5, ligne 33 - page 6, ligne 6; figures 2,3 *<br><br>-- | 5,6 |
| A | FR - A - 2 340 079 (IMPERIAL CHEMICAL INDUSTRIES LTD)<br><br>* Page 2, lignes 10-36 *<br><br>---- | 1,4 |

## DOCUMENTS CONSIDERES COMME PERTINENTS

**CLASSEMENT DE LA DEMANDE (Int. Cl. 3)**

A 61 F 1/00

**DOMAINES TECHNIQUES RECHERCHES (Int. Cl. 3)**

A 61 F
A 61 M

**CATEGORIE DES DOCUMENTS CITES**

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention
E: document de brevet antérieur, mais publié à la date de dépôt ou après cette date
D: cité dans la demande
L: cité pour d'autres raisons

&: membre de la même famille, document correspondant

Le présent rapport de recherche a été établi pour toutes les revendications

| Lieu de la recherche | Date d'achevement de la recherche | Examinateur |
|---|---|---|
| La Haye | 18-05-1982 | BARTLETT |